# EUROPEAN PATENT APPLICATION

(11) **EP 1 271 140 A1**
(43) Date of publication of application: **02.01.2003**
(21) Application number: 01202438.6
(22) Date of filing: 25.06.2001
(51) Int. Cl.: G01N 33/02

(54) **Determination of in-mouth sensory properties of foodstuffs**

(71) Applicant: Stichting Top-Instituut Voedselwetenschappen, 6700 AN Wageningen (NL)
(72) Inventor: Paques, Marcel, 3941 DM Doorn (NL); Weenen, Hugo, 1261 RV Blaricum (NL); Engelen, Lina, 6438 HB Nijmegen (NL); Van Riel, Johannes Antonius Maria, 6717 TM Ede (NL); Hamer, Robert Jan, 3813 RS Amersfoort (NL)
(74) Representative: Jorritsma, Ruurd

(57) **Abstract**

The present invention is concerned with a method of predicting in-mouth sensory properties of water-continuous foodstuffs that contain a dispersed lipid phase. The present method may advantageously be employed in product development and quality control.

More particularly the invention relates to a method of predicting in-mouth sensory properties of water-continuous foodstuffs that contain a dispersed lipid phase, said method comprising the following consecutive steps:
i. subjecting a sample of the foodstuff to in-mouth mastication or to conditions similar to those prevailing in the mouth during consumption of such a foodstuff,
ii. collecting at least a fraction of the sample for analysis,
iii. analysing the spatial distribution of the dispersed lipid phase within the collected fraction,
iv. determining from said analysis the extent to which the dispersed phase has migrated to the surface of the foodstuff as a result of step i. and
v. translating the results of the determination into predicted in-mouth sensory properties of the foodstuff.

## Description

### TECHNICAL FIELD

The present invention is concerned with a method of predicting in-mouth sensory properties of foodstuffs, more particularly the in-mouth sensory properties of water-continuous foodstuffs that contain a dispersed lipid phase. The present method comprises the steps of subjecting a sample of the foodstuff to conditions of mastication, determining the extent to which the dispersed lipid phase has migrated to the foodstuff surface and translating the results into in-mouth sensory properties of the foodstuff.

### BACKGROUND OF THE INVENTION

The in-mouth sensory properties of a foodstuff are without any doubt its most critical features, largely determining the extent in which such a foodstuff is liked or disliked by consumers. The in-mouth sensory properties of foodstuffs include a variety of attributes such as mouthfeel, creaminess, dispersibility, chewiness, flavour release, etc.

Because the in-mouth sensory properties are so important, a lot of the R&D work in relation to foodstuffs is aimed at optimising the in-mouth sensory properties. Mostly this is done by repeatedly going through the following optimisation loop:
a. Choose (new) product composition and (new) processing conditions
b. Produce samples on a pilot plant, using this composition and set of conditions
c. Subject the samples to an evaluation panel who rate the overall product and score the product on specific sensory features
d. Evaluate the results of c., also in the light of results obtained from previous cycles. If the results are not satisfactory, move back to step a.

In order to obtain meaningful results from product evaluations, the panels employed in product optimisation studies are usually quite big. For instance, it is not uncommon to have a panel of more than 10 people. Obviously, to train, set up and maintain such panels is laborious and costly. In particular panels used to quantitatively describe sensory attributes, such as Quantitative Descriptive Analysis panels, are quite expensive to operate.

Hence there is a need for food product evaluation methods which are less laborious and less expensive to operate than product evaluation panels and which produce results which provide a reliable measure of relevant in-mouth sensory properties of the food product. Ideally such an evaluation method would have to be an instrumental method, i.e. a method using devices rather than people to produce the desired results. In addition, such a method would be highly advantageous if it could be operated highly reproducibly and at high throughput.

### SUMMARY OF THE INVENTION

It was found that the aforementioned objectives can be realised by a method that is the subject of this application. The instrumental method according the present invention was developed following the discovery that in-mouth sensory properties of water-continuous foodstuffs containing a dispersed lipid phase are strongly related to the behaviour of the dispersed phase during mastication. It was surprisingly found that there is a very good correlation between the observed extent of migration of the lipid phase to the surface of a lipid-in-water type of foodstuff (as a result of mastication) and in-mouth sensory properties of the same foodstuff.

Starting from the above finding a method was developed that makes it possible to predict the in-mouth sensory properties of lipid-in-water type foodstuffs, which method employs a number of steps, including the step of subjecting a sample of the foodstuff to conditions of mastication, followed by the determination of the extent to which the dispersed lipid phase has migrated to the surface of the foodstuff and subsequently the translation of the results of the determination into a prediction of the in-mouth sensory properties of the foodstuff.

### DETAILED DESCRIPTION OF THE INVENTION

Hence one aspect of the invention is concerned with a method of predicting in-mouth sensory properties of water-continuous foodstuffs that contain a dispersed lipid phase, said method comprising the following consecutive steps:
i. subjecting a sample of the foodstuff to in-mouth mastication or to conditions similar to those prevailing in the mouth during consumption of such a foodstuff,
ii. collecting at least a fraction of the sample for analysis,
iii. analysing the spatial distribution of the dispersed lipid phase within the collected fraction,
iv. determining from said analysis the extent to which the dispersed phase has migrated to the surface of the foodstuff as a result of step i. and
v. translating the results of the determination into predicted in-mouth sensory properties of the foodstuff.

The present method is particularly suitable for predicting in-mouth sensory properties of water-continuous foodstuffs that contain a dispersed lipid phase, i.e. foodstuffs that can be characterised as lipid-in-water emulsions, water-in-lipid-in-water emulsions or bi-continuous lipid-and-water emulsions. Preferably the current method is applied to foodstuffs based on lipid-in-water emulsions or water-in-lipid-in-water emulsions.

The analysis of the spatial distribution of the dispersed lipid phase and the extend to which the dispersed phase has migrated to the surface as a result of the application of mastication conditions, may suitably be performed by means of a microscopic technique. Preferably the microscopic technique employed in the present method is selected from the group consisting of confocal scanning laser microscopy (CSLM), Raman-CSLM, light microscopy (in particular observation in differential interference contrast, or traditional wide field illumination of sections from embedded sample material), fluorescence microscopy, cryo-scanning electron microscopy (combination of secondary electron and back-scatter imaging of fracture surfaces and the outer surface of the sample material), transmission electron microscopy (observation of freeze fracture replica's, or thin sections of freeze substituted material followed by resin embedding), cryo-transmission electron microscopy (of cryo sections from -rapidly- frozen sample material) and environmental scanning electron microscopy (observation of the outer surface of the sample material).

Very good results in terms of predictive reliability are obtained with the present method if the time period lapsed between collecting the fraction and analysing the spatial distribution of the dispersed lipid phase is less than 15 minutes, preferably less than 5 minutes and more preferably less than 2 minutes. If the time lapsed between sample collection and analysis is very long, the results of the analysis will be less representative of the behaviour of the foodstuff during mastication. Hence it is desirable to minimise the duration of this period. Alternatively the sample may be "immobilised" by techniques known in the art within the aforementioned time periods so as to allow for analysis at a later time, without the aforementioned drawbacks.

The extent of migration observed in the present method may be determined visually or by means of devices that are able to automatically determine the spatial distribution of the dispersed lipid phase, or that can generate parameters that are indicative of said spatial distribution. In a preferred embodiment of the invention the extent of migration is determined by establishing the proportion of volume or surface-area occupied by the lipid phase within the exterior layer of the collected fraction as well as within another part of the same collected fraction, and by subsequently comparing the results. Most preferably the extent of migration is determined automatically, i.e. not visually, by means of a device capable of measuring the spatial distribution of the dispersed lipid phase.

When determining the extent of migration in accordance with the present method, it is advantageous to compare the proportion of volume or surface-area occupied by the lipid phase within an exterior layer of a thickness between 1 and 25 microns with the same proportion in another part of the same collected fraction. The thickness of the exterior layer to be applied for a certain foodstuff depends on the characteristics of said product. If the product is easily dispersed in the mouth and contains little or no gelling structures, a smaller layer can be applied than is the case for foodstuffs which do not disperse easily and that contain a strongly gelled aqueous phase. Usually the thickness of the exterior layer to be applied for a certain type of food product can easily be determined by viewing a sample subjected to steps i and ii of the present method using one of the microscopic techniques mentioned above. The image so obtained will normally reveal a layer at the surface of the sample which contains a relatively high density of lipid phase and an interior which contains a much lower density of dispersed lipid phase. The thickness of the exterior layer, as defined above, is to be chosen such that it encompasses the visually identifiable layer of accumulated lipid phase at the surface of the foodstuff. Naturally, when comparing the proportion of volume or surface-area occupied by the lipid phase inside and outside the layer, care should be taken to select areas for this comparison which are representative of the proportion found in the exterior layer as well as in the interior of the product.

In some cases foodstuffs may be submitted successfully to the present method without the need of any pre-treatment, e.g. to make it easier to distinguish the dispersed lipid phase from the continuous aqueous phase. Usually, however, it is easier to determine the extent to which the dispersed lipid phase has migrated by subjecting a sample of the foodstuff to a special pre-treatment prior to step i. so as to make the dispersed fat phase clearly distinguishable. In this respect the application of colouring techniques was found to be particularly advantageous.

As stated herein before it is a specific objective of the present invention to present a method of predicting in-mouth sensory properties of foodstuffs which method is less laborious and less expensive to operate than product evaluation panels and which, in addition, produces reliable results reproducibly and at high throughput. It was found that all of the aforementioned objectives are best achieved if in the present method step i. employs a mouth analogue rather than *in vivo* in-mouth mastication. Here the term "mouth analogue" relates to devices in which a sample of a foodstuff can be subjected to conditions that resemble the conditions prevailing in the mouth during consumption of such a foodstuff. Mouth analogues of various configurations are known in the art. Generally these mouth analogues have in common that they submit samples inserted therein to conditions of shear and heat exchange. Also they normally allow for the addition of (synthetic) saliva.

In a preferred embodiment of the present method step i. comprises inserting the sample into a mouth analogue wherein, for a period of between 2 seconds and 5 minutes, the sample is subjected to conditions of shear and kept in contact with a heat exchanging surface whose temperature is maintained at between 30 and 40 °C. In addition step i. may advantageously comprise the act of contacting the sample with an aqueous solution of amylase. The use of a mouth analogue in the fashion described here, makes it possible to obtain results more reproducibly and faster than is possible with *in vivo* in-mouth mastication by individuals. Naturally the application of mouth analogues, in particular if the procedure is automated, is much less laborious than the use of *in vivo* mastication.

The present method, especially when using a mouth analogue, is particularly suitable for screening large numbers of samples. It was surprisingly found that when gas, e.g. air, is bubbled through the sample, the rate at which the dispersed phase moves to the surface of the foodstuff is increased substantially. Thus, by bubbling a gas through the sample, it is possible to significantly reduce the time needed for performing step 1. of the present method, making the method even more suitable for high throughput screening. Hence in a preferred embodiment of the invention, step 1. comprises inserting the sample into a mouth analogue wherein gas, preferably air, is bubbled through the sample. The gas may suitably be bubbled through the sample for a period of time ranging from 2 seconds to 3 minutes. The amount of air bubbled through the sample will preferably exceed 0.02 ml per gram of sample per second.

In principle the method of the invention may be used to predict a variety of in-mouth sensory properties. The method was found to be particularly useful and reliable when used to predict the mouthfeel, and more particularly the creaminess of the foodstuff upon consumption. Here the terms "mouthfeel" and "creaminess" relate to product attributes which may be described as follows. Creaminess: soft, smooth and velvety impression felt all over the inside of the mouth during and after eating a food; it is generally associated with fat, and appears to contain textural and flavour components. Mouthfeel: sensations in the mouth not directly caused by interaction of a food with nasal flavour and/or oral taste buds.

Another sensory attribute which can be predicted reliably with the present method is in-mouth flavour release. It was found that flavour intensity, i.e. rate of flavour release is positively correlated with the extent of migration observed in the present method.

The advantages of the present method are particularly profound if the method is used in a process of developing water and lipid containing foodstuffs wherein the in-mouth sensory properties of the foodstuff are optimised on the basis of the results obtained from the method according to the invention. As described above such product optimisation usually involves many cycles of sample preparation, evaluation, adjustment and repeated sample preparation. The present invention makes it possible to bring down the time and/or effort needed to complete such cycles.

In another advantageous embodiment of the invention the results obtained from the present predictive method are used in a process of routinely determining the quality of water and lipid containing foodstuffs. The present method can, for instance, suitably be used as a quality control and/or quality assessment tool. The present method offers the advantage that is faster, cheaper and more accurate than panel evaluations applied for the same purpose. Also it provides more reliable results than any known analytical techniques that have so far been developed for the same purpose.

Another aspect of the invention relates to the use of microscopy in a method of predicting in-mouth sensory properties of foodstuffs containing a dispersed phase, said method comprising the steps of:
i. subjecting a sample of the foodstuff to in-mouth mastication or to conditions similar to those prevailing in the mouth during consumption of such a foodstuff,
ii. determining by means of microscopy to which extent the dispersed phase has migrated to the foodstuff-air interface as a result of step i.
The invention is further illustrated by, but not limited to the following examples.

### EXAMPLE

This example describes a method for predicting the "Creaminess" of dairy custards by analysing their microstructure with the help of confocal scanning laser microscopy.

### CSLM instrumentation and observation conditions

Observations were done in a LEICA TCS SP Confocal Scanning Laser Microscope (CSLM), in single photon mode, configured with an inverted microscope (model Leica DM IRBE), and using an Ar/Kr laser. The following Leica objective lenses were used: 20x/0.7NA/dry/HC PL APO, 63x/UV/1.25NA/water immersion/PL APO. Fluorescent probes used were FITC (fluorescein-5-isothiocyanate) and Nile Red. The excitation wavelengths were 488 and 568 nm, respectively, and the emission wavelengths were at 525 and 525-605 nm. Observation was done at the outer surface of the product at locations away from the surface of the cover glass, to prevent any effects from possible glass/product interactions. Digital image files were acquired in multiple .tif format and in 1024x1024-pixel resolution.

### Non-covalent labelling

A mixture of FITC with Nile Red was used for non-covalent labelling. A small piece of sintered glass containing in its pores an amount of two drops of a 0.1% FITC and 0,1% Nile Red in polyethylene glycol-glycerol solution was added to 2 ml of sample. The probe molecules will spread from the pores of the sintered glass over the sample according to local accessibility and affinity. The applied probes show different affinities for hydrophilic and hydrophobic domains. The FITC molecules accumulate in hydrophilic regions and the Nile Red molecules accumulate in hydrophobic regions. Consequently, structural elements will be highlighted in a way that is dominated by the kinetics and thermodynamics of the probe molecules in the specimen matrix.

### Mouth processing "Tonguer" method

The tongue is moved back, forth and sideways against the palate, moving the dairy custard around as well.

### Experiments

Directly following taking the untreated dairy custard out of the refrigerator (storing temperature approximately 5 C) a sample was taken and prepared for observation. The total time between taking the sample from the refrigerator and actual observation in the CSLM was less than 1 minute. For "mouth processing" a spoonful of product was taken directly from the refrigerator and put into the mouth. After approximately 10 to 15 sec. of mouth processing the product was spitted out into a small container. A sample volume of 1.5 ml was transferred into a small eppendorf vessel, on top of 10 µL FITC/NileRed solution, followed by very mild and gentle mixing. From the stained product a very small sample was taken and transferred onto a cover glass followed by observation in the microscope. The total time between taking the sample from the refrigerator and actual observation in the CSLM was less then 1 minute.

### Results

The oral processing results in a sequence of microstructural changes of the product. The continuous aqueous starch phase (grey in the Figures) was broken down resulting in a considerable drop of viscosity. The viscosity drop allows free mobility of the fat globules (black spherical particles in the Figures). As a result of physical factors during mouth processing the fat globules redistribute from their original heterogeneous scattered locations over the bulk to a thin layer at the outer surface of the product where they accumulate ("oil droplet surfacing effect"). The accumulation in the layer at the outer surface is followed by coalescence and exudation of the oil droplets from the continuous aqueous phase onto the outer surface of the product sample creating a covering layer of a continuous oil phase. Below the mains findings are illustrated by two CSLM images. The original microstructure is represented in fig. A, and the microstructure after oral processing in fig. B. In order to facilitate black-and-white printing, the colour images obtained were converted into images using grey shades. Because of some small differences in the nature of the 2 images it was decided to apply slightly different image processing procedures for both images. As a result the air phase in figure B is white, whereas in figure A it is black.

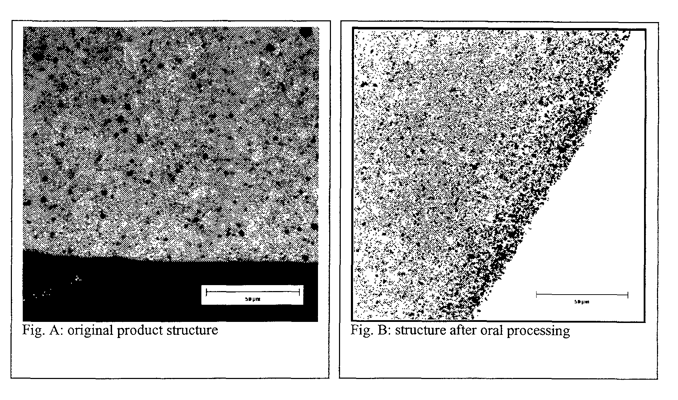

## Claims

1. Method of predicting in-mouth sensory properties of water-continuous foodstuffs that contain a dispersed lipid phase, said method comprising the following consecutive steps:
i. subjecting a sample of the foodstuff to in-mouth mastication or to conditions similar to those prevailing in the mouth during consumption of such a foodstuff,
ii. collecting at least a fraction of the sample for analysis,
iii. analysing the spatial distribution of the dispersed lipid phase within the collected fraction,
iv. determining from said analysis the extent to which the dispersed phase has migrated to the surface of the foodstuff as a result of step i. and
v. translating the results of the determination into predicted in-mouth sensory properties of the foodstuff.

2. Method according to claim 1 wherein the analysis is performed by means of a microscopic technique, preferably a microscopic technique selected from the group consisting of confocal scanning laser microscopy (CSLM), Raman-CSLM, light microscopy, fluorescence microscopy, cryo-scanning electron microscopy, transmission electron microscopy, cryo-transmission electron microscopy and environmental scanning electron microscopy.

3. Method according to claim 1 or 2, wherein the time period lapsed between collecting the fraction and analysing the spatial distribution of the dispersed lipid phase is less than 15 minutes, preferably less than 5 minutes, more preferably less than 2 minutes.

4. Method according to any one of claims 1-3, wherein the extent of migration is determined by establishing the proportion of volume or surface-area occupied by the lipid phase within the exterior layer of the collected fraction as well as within another part of the same collected fraction, and by subsequently comparing the results.

5. Method according to any one of claims 1-4, wherein step i. comprises inserting the sample into a mouth analogue wherein, for a period of between 2 seconds and 5 minutes, the sample is subjected to conditions of shear and kept in contact with a heat exchanging surface whose temperature is maintained at between 30 and 40 °C.

6. Method according to claim 5, wherein step i. comprises contacting the sample with an aqueous solution of amylase.

7. Method according to any one of claims 1-6, wherein the method is used to predict the mouthfeel, more particularly the creaminess of the foodstuff upon consumption.

8. Process of developing water and lipid containing foodstuffs wherein the in-mouth sensory properties of the foodstuff are optimised on the basis of the results obtained from the method according to any one of claims 1-7.

9. Process of routinely determining the quality of water and lipid containing foodstuffs wherein the quality of the foodstuff is determined on the basis of the results obtained from the method according to any one of claims 1-7.

10. Use of microscopy in a method of predicting in-mouth sensory properties of foodstuffs containing a dispersed phase, said method comprising the steps of:
i. subjecting a sample of the foodstuff to in-mouth mastication or to conditions similar to those prevailing in the mouth during consumption of such a foodstuff,
ii. determining by means of microscopy to which extent the dispersed phase has migrated to the foodstuff-air interface as a result of step i..
